(19) Europäisches Patentamt
European Patent Office
Office européen des brevets

(11) Veröffentlichungsnummer: **0 266 719 A1**

(12) # EUROPÄISCHE PATENTANMELDUNG

(21) Anmeldenummer: **87116074.3**

(51) Int. Cl.[4]: **C07J 5/00**

(22) Anmeldetag: **02.11.87**

(30) Priorität: **06.11.86 DE 3637806**

(43) Veröffentlichungstag der Anmeldung: **11.05.88 Patentblatt 88/19**

(84) Benannte Vertragsstaaten: **AT BE CH DE ES FR GB GR IT LI LU NL SE**

(71) Anmelder: **HOECHST AKTIENGESELLSCHAFT**
**Postfach 80 03 20**
**D-6230 Frankfurt am Main 80(DE)**

(72) Erfinder: **Martin, Wolfgang, Dr.**
**Am Schieferberg 19**
**D-6233 Kelkheim (Taunus)(DE)**

(54) **Verfahren zur Herstellung von Prednisolon-17-ethylcarbonat durch Umlagerung von Prednisolon-21-ethylcarbonat.**

(57) Beschrieben wird ein Verfahren zur Herstellung von (Methyl)-Prednisolon-17-($C_1$-$C_4$)carbonat durch Umlagerung von (Methyl)-Prednisolon-21-($C_1$-$C_4$)carbonat

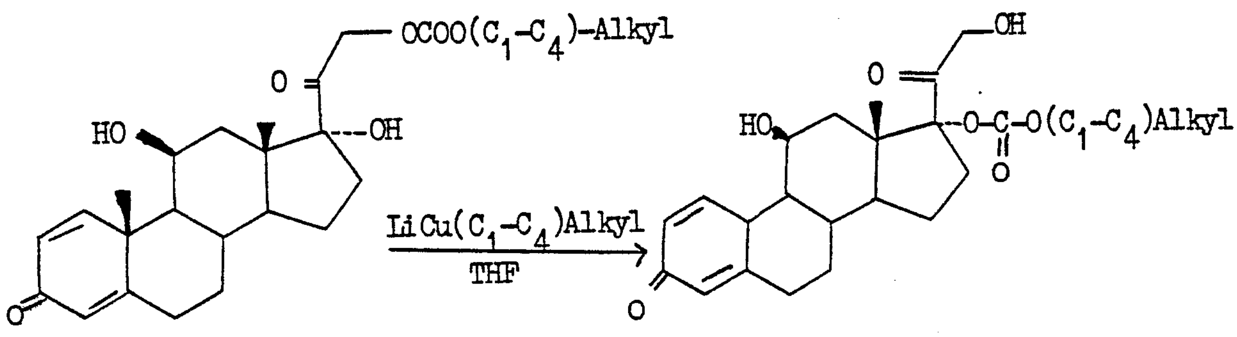


nach welchem man Prednisolon-bzw. Methylprednisolon-21-($C_1$-$C_4$)alkylcarbonat mittels Lithiumdi-($C_1$-$C_4$)-alkylcuprat umlagert.

EP 0 266 719 A1

## Verfahren zur Herstellung von Prednisolon-17-ethylcarbonat durch Umlagerung von Prednisolon-21-ethylcarbonat

Die Erfindung ist gerichtet auf ein Verfahren zur Herstellung von (Methyl)-Prednisolon-17-($C_1$-$C_4$)-alkylcarbonat aus (Methyl)-Prednisolon-21-($C_1$-$C_4$)alkylcarbonat.

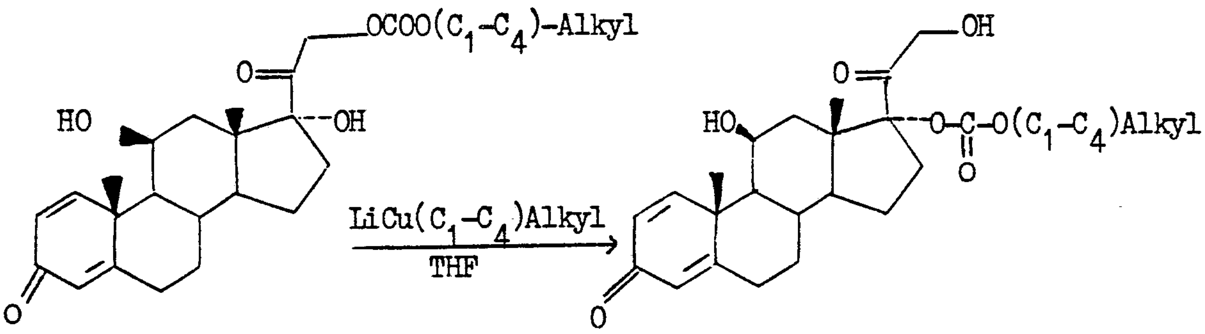


Prednisolon-17-ethylcarbonat ist ein wichtiges Zwischenprodukt bei der Synthese des im EP 742 bzw. US 4242334 beschriebenen Prednisolon-17-ethylcarbonat-21-propionats.

Bekanntlich ist die selektive Veresterung von Prednisolon an der 17-OH-Gruppe auf direktem Weg nicht möglich, da stets die 21-OH-Gruppe zuerst reagiert.

In den obengenannten Patenten ist die Herstellung der Titelverbindung über ein zyklisches Prednisolon-diethylorthocarbonat beschrieben, welches anschließend sauer gespalten wird. Nachteile des Verfahrens sind der enorm hohe Preis des hierbei benötigten Tetraethylorthocarbonat-Reagenzes und die teilweise Rückspaltung des zyklischen Prednisolonorthocarbonats zu Prednisolon sowie die unerwünschte Spaltung und Umesterung unter den Spaltbedingungen zu Prednisolon-21-ethylcarbonat.

Entsprechend der vorliegenden Erfindung wird Methylprednisolon-bzw. Prednisolon-21-($C_1$-$C_4$)-alkylcarbonat, das durch Umsetzung von Methylprednisolon bzw. Prednisolon mit Chlorameisensäureethylester kostengünstig und mit praktisch quantitativer Ausbeute herstellbar ist, mit Lithiumdi-($C_1$-$C_4$)alkylcuprat in Tetrahydrofuran in (Methyl)-Prednisolon-17-ethylcarbonat umgelagert.

Im EP 72546 ist eine ähnliche Umlagerung beschrieben, jedoch ist diese ganz speziell für 21-Alkoxymethylcarbonsäureester, jedoch nicht für Alkylcarbonate von Trihydroxypregnendion bzw. -pregnadiendion abgefaßt. Es war nicht zu erwarten, daß die Reaktion mit Alkylcarbonaten ebenso oder gar besser ablaufen würde, als mit Alkoxycarbonsäureestern. (Die meisten in EP 72546 angegebenen Umlagerungsausbeuten liegen unter 50 %, gegenüber 67 - 81 % bei Prednisolon-17-ethylcarbonat.)

Die Umlagerung des Methylprednisolon-bzw. Prednisolon-21-($C_1$-$C_4$)-alkylcarbonats in das Methylprednisolon-bzw. Prednisolon-17-($C_1$-$C_4$)-alkylcarbonat erfolgt in der Weise, daß man eine Lösung des Methylprednisolon-bzw. Prednisolon-21-($C_1$-$C_4$)alkylcarbonats in Tetrahydrofuran zu einer aus Kupfer(I)jodid und ($C_1$-$C_4$)-Alkyllithium in Diethylether hergestellten Lithiumdi-($C_1$-$C_4$)alkylcupratlösung in Tetrahydrofuran zugibt und die resultierende Suspension in wässriges Ammoniumchlorid einfließen läßt. Aus der organischen Phase und aus den Methylenchloridextrakten wird die gesuchte Verbindung, die aus Ethanol kristallisierbar ist, in ca. 95 %iger Reinheit erhalten.

Bevorzugt wird für die Umlagerung des 21-Alkylcarbonats in das entsprechende 17-Alkylcarbonat Methyllithium oder Butyllithium verwendet.

Die Umlagerung wird bei einer Temperatur von unterhalb +30°C, vorzugsweise unterhalb 0°C, insbesondere unterhalb -20°C, aber oberhalb von -50°C, vorzugsweise oberhalb von -40°C, insbesondere bei ca. -30°C durchgeführt.

Die Reaktionsdauer schwankt zwischen wenigen Minuten und 5 Stunden, insbesondere zwischen einer halben und einer Stunde.

Beispiel 1: Prednisolon-21-ethylcarbonat (Ausgangsmaterial)

36 g (0,1 Mol) Prednisolon werden in 150 ml Methylenchlorid suspendiert und bei Zimmertemperatur mit 13,8 ml Triethylamin und 9,56 ml Chlorameisensäureethylester versetzt. Nach dem Abklingen der exothermen Reaktion rührt man 1 - 2 Std. bei 40°C, fügt danach noch zweimal je 7 ml Triethylamin und 4,8 ml Chlorameisensäureethylester zu und rührt so lange bei 40°C, bis ein Umsatz von wenigstens 96 % (HPLC) erreicht ist. Nach Entfernen des Lösungsmittels im Vakuum wird der Rückstand aus 50 ml Ethanol kristallisiert.
Ausbeute: 44,2 g (Gehalt: 97,3 %) = 98 % d.Th.

Beispiel 2: Prednisolon-17-ethylcarbonat

6,4 g (0,0336 Mol) Kupfer(I)jodid werden bei 0°C in 128 ml trockenem Tetrahydrofuran mit 40 ml einer 5 %igen Lösung von Methyllithium in Diethylether (ca. 0,068 Mol) versetzt. Die resultierende klare Lösung wird auf -30°C abgekühlt und bei dieser Temperatur mit einer Lösung (teilweise Suspension) von 5,8 g Prednisolon-21-ethylcarbonat (97,3 %ig; 0,013 Mol) in 200 ml Tetrahydrofuran von 20°C während 25 Minuten versetzt. Die resultierende gelbe Suspension wird noch 20 min bei -30°C gerührt und danach in eine Lösung von 20 g Ammoniumchlorid in 200 ml Wasser gegossen.

Die organische Phase wird abgetrennt, im Vakuum auf ein Drittel eingeengt und in 150 ml Methylenchlorid aufgenommen. Die Wasserphase wird mit insgesamt 300 ml Methylenchlorid extrahiert; der Extrakt wird mit 100 ml Wasser, 100 ml 10 %iger wässriger Ammoniumchloridlösung und danach mit 100 ml Wasser gewaschen. Die Methylenchloridphasen werden vereinigt, über Natriumsulfat getrocknet und im Vakuum zu einem glasigen Schaum eingeengt.
Ausbeute roh: 4,8 g (HPLC: 95,2 % Prednisolon-17-ethylcarbonat, 2,8 % Prednisolon-21-ethylcarbonat, 0,5 % Prednisolon) = 81 % d.Th. Roh-Ausbeute: nach Kristallisation aus 50 ml Ethanol: 4,0 g (HPLC: 95,0 % Prednisolon-17-ethylcarbonat, 2,8 % Prednisolon-21-ethylcarbonat, 2,1 % Prednisolon) = 67 % d.Th.

Der Reinheitsgrad des Prednisolon-17-ethylcarbonats kann durch einfache Umkristallisation aus Ethanol noch gesteigert werden.

Beispiel 3: Prednisolon-17-ethylcarbonat

12,8 g Kupfer(I)jodid werden bei 0°C in 256 ml absolutem Tetrahydrofuran suspendiert und bei dieser Temperatur unter einer Stickstoffatmosphäre mit 82 ml einer Butyllithiumlösung (15 % in n-Hexan) während 10 Minuten versetzt.

Man rührt noch 5 Minuten bei 0°C, kühlt auf -30°C ab und tropft danach eine Lösung von 11,7 g Prednisolon-21-ethylcarbonat, hergestellt aus 8,97 g Prednisolon nach Beispiel 1, in 400 ml absolutem Tetrahydrofuran während 30 Minuten bei -30°C zu.

Man rührt noch 5 Minuten, zersetzt durch Eingießen in eine Lösung von 40 g Ammoniumchlorid in 400 ml Wasser, rührt 5 Minuten, trennt die Phasen, engt die Organophase auf ca. ein Drittel des ursprünglichen Volumens ein, extrahiert die Wasserphase mit 2 x 100 ml Methylenchlorid, vereinigt die eingedampfte Organophase mit den Methylenchloridextrakten, filtriert vom ausgefallenen Kupfer(I)jodid ab, wäscht das Filtrat mit 150 ml einer 10 %igen Ammoniumchloridlösung und 150 ml Wasser, trocknet über Natriumsulfat, engt ein und kristallisiert aus 30 ml Ethanol. Ausbeute: 4,32 g Prednisolon-17-ethylcarbonat (HPLC: 91,0 %ig) ≙ 36 % d.Th. bez. auf Prednisolon.

## Ansprüche

1. Verfahren zur Herstellung von Prednisolon-bzw. Methylprednisolon-17-($C_1$-$C_4$)alkylcarbonat, dadurch gekennzeichnet, daß man Prednisolon-bzw. Methylprednisolon-21-($C_1$-$C_4$)alkylcarbonat mittels Lithiumdi-($C_1$-$C_4$)alkylcuprat umlagert.

2. Verfahren nach Anspruch 1, dadurch gekennzeichnet, daß man die Umlagerung mit Lithiumdimethyl- oder Lithiumdibutylcuprat durchführt.

Europäisches Patentamt

# EUROPÄISCHER RECHERCHENBERICHT

Nummer der Anmeldung

EP 87 11 6074

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | DE-A-2 340 591 (GLAXO LABORATORIES LTD)<br>* Ansprüche 1-5,13,14,19 * | 1,2 | C 07 J 5/00 |
| Y | * Seiten 22-25, Tabelle III * | 1 | |
| D,Y | EP-A-0 072 546 (SCHERING AG)<br>* Anspruch 25; Beispiel 1b * | 1 | |
| P,Y | EP-A-0 000 742 (HOECHST AG)<br>* Ansprüche; Seiten 52,53; Beispiel 29 * | 1 | |
| Y | ARZNEIMITTEL-FORSCHUNG, Band 35, Nr. 12, Dezember 1985; Seiten 1753-1757, Aulendorf, DE; U. STACHE et al.: "Synthese von Prednicarbat, einem halogenfreien, topisch antiinflammatorisch wirksamen Derivat des Prednisolon-17-ethylkohlensäureesters"<br>* Seiten 1754,1756 * | 1 | |

| RECHERCHIERTE SACHGEBIETE (Int. Cl.4) |
|---|
| C 07 J 5/00 |

Der vorliegende Recherchenbericht wurde für alle Patentansprüche erstellt

| Recherchenort | Abschlußdatum der Recherche | Prüfer |
|---|---|---|
| DEN HAAG | 08-02-1988 | HENRY J.C. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
& : Mitglied der gleichen Patentfamilie, übereinstimmendes Dokument

EPO FORM 1503 03.82 (P0403)